# EUROPÄISCHE PATENTSCHRIFT

(11) **EP 1 359 151 B1**
(45) Veröffentlichungstag und Bekanntmachung des Hinweises auf die Patenterteilung: **03.06.2015**
(21) Anmeldenummer: 03009385.0
(22) Anmeldetag: 30.11.2001
(51) Int. Cl.: C07D 295/023

(54) **Verfahren zur Herstellung von Triethylendiamin (TEDA)**
Process for the preparation of triethylenediamine (TEDA)
Procédé de préparation de la triéthylène diamine (TEDA)

(30) Priorität: 12.12.2000 DE 10061863
(43) Veröffentlichungstag der Anmeldung: 05.11.2003
(62) Teilanmeldung aus: 01128600.2
(73) Patentinhaber: BASF SE, 67056 Ludwigshafen (DE)
(72) Erfinder: Frauenkron, Matthias, Dr., 67251 Freinsheim (DE); Stein, Bernd, Dr., 64665 Alsbach-Hähnlein (DE)

(56) Entgegenhaltungen:
- EP-A- 0 158 319
- EP-A- 0 263 463
- EP-A- 0 312 734
- EP-A- 0 313 753
- WO-A-98/55228
- US-A- 3 702 886
- US-A- 3 956 329
- DATABASE CA [Online] CHEMICAL ABSTRACTS SERVICE, COLUMBUS, OHIO, US; HU, XIAO ET AL: "Synthesis of triethylenediamine using modified zeolite as catalyst" retrieved from STN Database accession no. 134:268039 XP002253039 & HUAGONG KEJI (2000), 8(3), 46-49 ,
- REICHLE WALTER T: "Reactions of aliphatic.alpha.-.omega.-diamines in H+-pentasils" JOURNAL OF CATALYSIS, ACADEMIC PRESS, DULUTH, MN, US, Bd. 144, Nr. 2, 1993, Seiten 556-568, XP002192171 ISSN: 0021-9517

## Beschreibung

Die vorliegende Erfindung betrifft ein Verfahren zur Herstellung von Triethylendiamin (TEDA) durch Umsetzung von Ethylendiamin (EDA) in Gegenwart eines Zeolith-Katalysators.

Triethylendiamin (TEDA = DABCO® = 1,4-Diazabicyclo-[2,2,2]-octan) ist ein wichtiger chemischer Grundstoff und findet unter anderem Verwendung bei der Herstellung von Pharmazeutika und Kunststoffen, insbesondere als Katalysator bei der Herstellung von Polyurethanen.

Die bekannten Verfahren zur Herstellung von TEDA unterscheiden sich im wesentlichen durch die Art der Ausgangsprodukte und der Katalysatoren. Grundsätzlich ist es vorteilhaft als Edukte günstige Basischemikalien, wie z.B. Monoethanolamin (MEOA) oder Ethylendiamin (1,2-Diaminoethan, EDA) einzusetzen. Herkömmliche Verfahren haben sich jedoch, insbesondere gegenüber dem Edukt EDA, als sehr wenig selektiv erwiesen. Überdies ist die Abtrennung der Verunreinigungen die bei der Cyclisierungsreaktion entstehen schwierig, so dass sich diese Verfahren technisch nicht durchsetzen konnten.

Das in der US 3,285,920 (H.G. Muhlbauer et al., Jefferson Chemical Co.) beschriebene Verfahren zur gleichzeitigen Herstellung von TEDA und Piperazin (im Folgenden PIP genannt) ist ein 2-Stufenprozess, nach dem man zunächst EDA, Ethanolamin und/oder deren Oligomere in Gegenwart von Ammoniak und Wasserstoff zu einem Gemisch aus Piperazin und N-(Beta-aminoethyl)-piperazin in einem reduktiven Aminierungsverfahren unter Einsatz von metalloxidischen Hydrierkatalysatoren umsetzt und den verbleibenden Rest- nach Abtrennung des Piperazins - in Gegenwart von Cyclisierungskatalysatoren wie Phosphatsalzen und Alumosilikaten cyclisiert. Die Ausbeuten an TEDA liegen bei etwa 25 %, die von PIP bei etwa 12 %.

US-A-2,937,176 (Houdry Process Corp.) betrifft die Herstellung von TEDA durch Gasphasenreaktion eines Alkylenpolyamins oder Alkanolamins in Gegenwart eines festen sauren Katalysators, wie Silica-Alumina, bei Temperaturen von 300 bis 500°C. Die Aufreinigung des TEDAs erfolgt durch Kristallisation aus Kohlenwasserstoffen, bevorzugt Pentan.

DE-A-24 34 913 (Shunan Petrochemicals) (Äquivalent: US-A-3,956,329) beschreibt den Einsatz von Pentasilzeolithen zur TEDA-Synthese aus Aminen wie N-Aminoethylpiperazin, PIP oder EDA mittels Umsetzung an Zeolithen des Typs A, X und Y der allgemeinen Formel a(M₂/ₙO) (Al₂O3) m(SiO₂) mit M = Alkali-, Erdalkalimetall, Element der Zinkgruppe, H⁺ oder NH₄⁺; n = Valenz des Kations; a = 1, 0 ± 0,5; n = 2 - 12. Die Zeolithe werden zur Überführung in die gewünschte Form mit einer wässrigen Lösung von Salzsäure zum Ionenaustausch mit Wasserstoffkationen bzw. mit Metallhalogeniden zum Ionenaustausch mit den gewünschten Metallkationen behandelt.

EP-A-158 319 (Union Carbide Corp.) betrifft die Herstellung von 1-Aza-bicyclo[2.2.2]octan und 1,4-Diaza-bicyclo[2.2.2]octanen aus acyclischen oder heterocyclischen Aminen in Gegenwart eines 'high-silica zeolite'-Katalysators.

Aus EP-A-313 753 (Äquivalent: DE-A1-37 35 212) und EP-A-312 734 (Äquivalent: DE-A1-37 35 214) (beide Hüls AG) ist ein Verfahren zur Herstellung eines PIP/TEDA Gemisches durch Umsetzung von Ethanolaminen und/oder Ethylendiamin in Gegenwart eines Zeolithen vom Pentasil-Typ bekannt. Nach dem Verfahren wird das Reaktionsgut bei 280 bis 380°C, einer LHSV (liquid hourly space velocity) von 0,1 bis 10 h⁻¹ und bei einem Absolutdruck von 0,1 bis 10 bar in gasförmiger Form über einen Festbettkatalysator geleitet. Es wird auch vorgeschlagen, die Ausgangsverbindungen zusammen mit einem Verdünnungsmittel, wie z. B. Wasser, einzusetzen. Es werden TEDA-Selektivitäten von max. 46 % erzielt.

Gemäß der EP-A-382 055 (Äquivalent: DE-A-39 03 622, BASF AG) werden 1,2-Diaminoethan (EDA) und 0 bis 200 mol% Piperazin an Aluminium-, Bor-, Gallium- und/oder Eisensilikat-Zeolithen bei folgenden bevorzugten Reaktionsbedingungen, im Falle einer Flüssigphasen-Reaktion, zu TEDA umgesetzt: Reaktionstemperatur 100 bis 300°C, Druck 1 bis 5 bar und WHSV 1 bis 10 h⁻¹. Bevorzugt soll die Reaktion in der Gasphase bei einer Reaktionstemperatur von 200 bis 400°C, einem Druck von 0,5 bis 5 bar und einer WHSV von 1 bis 10 h⁻¹ durchgeführt werden. Ein Lösungs- bzw. Verdünnungsmittel wie Wasser kann zugesetzt werden. In der bevorzugten Gasphasenfahrweise werden Ausbeuten an TEDA von bis zu 70 % erhalten. Als besondere Präparationsweise wird nach der Verformung der Zeolithe eine Behandlung mit wässriger Salzsäure und anschließender Kalzinierung bei 400 bis 500°C beschrieben.

EP-A-423 526 (Äquivalent: DE-A-39 34 459, Bayer AG) beschreibt ein Verfahren zur Herstellung von TEDA und PIP durch Umsetzung von EDA an Zeolithen vom Pentasil-Typ mit abgeschwächter Acidität. Solche Zeolithe sind nach dieser Anmeldung durch Austausch von mindestens 50 % aller austauschbaren Kationen durch Alkalimetallkationen erhältlich oder sind solche, bei denen das Aluminium des Zeolith-Gerüstes isomorph durch Eisen ersetzt ist. Nicht nach diesen Verfahren behandelte ZSM-5 Katalysatoren haben sich gemäß dieser Anmeldung als weniger geeignet erwiesen. Die Umsetzung wird bei einer Temperatur von 300 bis 400°C und bei einer Katalysatorbelastung von 0,03 bis 2,0 kg (EDA) / kg (Zeolith) / h durchgeführt, wobei EDA / Wasser-Gemische mit 2 bis 25 Mol, vorzugsweise 5 bis 15 Mol, Wasser pro Mol EDA eingesetzt werden. Es werden Selektivitäten bezüglich TEDA von bis zu 65 % erzielt.

US-A-4,966,969 (Idemitsu Kosan) beschreibt eine Methode zur Herstellung von TEDA aus aminhaltigen Verbindungen, wie z.B. Monoethanolamin, Ethylendiamin, Piperazin oder Piperazinderivaten, an Metallsilicaten des Pentasil-Typs mit SiO₂Al₂O₃-Verhältnissen von über 12, die bei 400 - 600°C unter Luft kalziniert worden, bei Reaktionstemperaturen von 100 - 500°C und Drücken ab 3 bar.

Im US-A-5,041,548 (Idemitsu Kosan Ltd.) wird unter anderem vorgeschlagen, Pentasil-Zeolithe (SiO₂/M₂O₃: z.B. H-ZSM5, SiO₂/Al₂O₃ = 45 - 90), die in Gegenwart organischer Template wie Tetraalkylammonium-Verbindungen hergestellt worden, bei der Umsetzung von aminhaltigen Verbindungen, wie z.B. Monoethanolamin, Ethylendiamin oder Piperazin, zur Herstellung von TEDA zu verwenden. Es werden bei der Umsetzung von EDA / Wasser-Gemischen bei 400°C TEDA-Ausbeuten von 45 % erreicht. Pentasil-Zeolithe, die ohne organisches Templat präpariert wurden, zeigen in den Umsetzungen der aminhaltigen Verbindungen bei 350 - 400°C wesentlich schlechtere TEDA-Ausbeuten.

In EP-A-831 096, EP-A-842 936 und EP-A-952 152 (Air Products and Chemicals Inc.) werden Verfahren zur Herstellung von TEDA aus EDA oder Monoethanolamin unter Verwendung von speziell modifizierten Pentasil-Zeolithen beschrieben:
Gemäß EP-A-831 096 (Äquivalent: US-A-5,731,449) kann durch eine Laugenbehandlung eines Pentasil-Zeolithen (Na-ZSM5, SiO₂/Al₂O₃ = 160), der anschließend mit NH₄NO₃-Lsg. und Kalzination in die H⁺-Form überführt wurde (H-ZSM5, SiO₂/Al₂O₃ = 153), eine Steigerung in der Selektivität bzgl. TEDA von 23 % auf 56 % und in der Langzeitstabilität auf 32 h ohne sichtbare Desaktivierung bei der Umsetzung eines EDA / Wasser-Gemisches bei 340°C gegenüber unbehandelten Zeolithen erreicht werden. Der Effekt wird durch die Passivierung der aktiven Zentren (Hydroxyl-Gruppen, Analyse mit IR-Spektroskopie) auf der externen, äußeren Oberfläche des Zeolithen infolge der Laugenbehandlung erklärt.
Gemäß EP-A-842 936 (Äquivalent: US-A-5,741,906) kann durch eine Vorbehandlung mit einem Dealuminierungsagens (Chelatbildner zur Entfernung von Aluminium, z.B. Oxalsäure) die externe, äußere Oberfläche von Pentasil-Zeolithen (H-ZSM5, SiO₂/Al₂O₃ = 180) ebenfalls passiviert werden und so beipsielsweise eine verbesserte Selektivität bei der TEDA-Synthese aus Monoethanolamin, Piperazin und Wasser bei 350°C von bis zu 30 % gegenüber unbehandelten Zeolithen erzielt werden.
Gemäß EP-A-952 152 (Äquivalent: US-A-6,084,096) kann eine Oberflächenpassivierung der Pentasil-Zeolithe ebenfalls durch die Behandlung mit einer Siliciumverbindung und anschließender Kalzination erreicht werden. Durch die Behandlung eines sehr fein kristallinen Pentasil-Zeolithen (H-ZSM5, SiO₂/Al₂O₃ = 90, Kristallgröße: 0,07 ∝m) mit einer Lösung aus Tetraethoxysilan in Ethanol und nachfolgender Kalzination konnte beispielsweise bei leichtem Rückgang der Aktivität für die Umsetzung eines EDA / Wasser-Gemisches bei 340°C die Selektivität bzgl. TEDA und PIP von 81 % auf 89 % gegenüber dem unbehandelten Material erhöht werden.
EP-A-842 935 (Äquivalent: US-A-5,756,741) (Air Products and Chemicals Inc.) beschreibt ein zweistufiges Verfahren, in dem zunächst aus einer Amino-Verbindung durch Cyclisierungsreaktion eine Piperazin-reiche Zwischenstufe hergestellt wird, die dann unter Zusatz von z.B. EDA an einem Pentasil-Zeolith zu TEDA umgesetzt wird. Diese spezielle zweistufige Fahrweise soll die Notwendigkeit einer PIP-Rückführung bei der TEDA-Synthese minimieren oder sogar eliminieren.
EP-A-1 041 073 (Tosoh Corp.) betrifft ein Verfahren zur Herstellung von Triethylendiaminen und Piperazinen durch Kontaktieren von bestimmten Verbindungen mit einer Aminoethyl-Gruppe mit einem kristallinen Aluminosilikat, in dem das Silica/Alumina- Verhältnis mindestens 12 beträgt. Das verformte Alumosilikat wird bei einer Temperatur von 500 - 950°C, bevorzugt bei 550 - 850°C, für mindestens eine (bevorzugt 3) Stunden kalziniert. Im Anschluss an die Kalzination erfolgt eine Säurebehandlung mit einer wässrigen anorganischen Säure bei 50 - 80°C für 3 bis 50 Stunden.
US-A-4,289,881 (Bayer AG; Äquivalent: EP-A-10 671) beschreibt die TEDA-Herstellung aus bestimmten Piperazinderivaten in Gegenwart eines SiO₂-Katalysators.
DD-A-206 896 (VEB Leuna-Werke) betrifft ein Verfahren zur Herstellung von TEDA durch Umsetzung von N-(beta-Aminoethyl)piperazin und/oder N-(beta-Hydroxyethyl)piperazin an einem porösen SiO₂/Al₂O₃-Katalysator in Gegenwart von NH₃.
Derwent Abstract Nr. 1997-371381 (RU-A-20 71 475 (AS Sibe Catalysis Inst.)) beschreibt die Herstellung von Triethylendiaminen aus Monoethanolamin an einem Pentasil-Zeolith, der mit einer wässrigen Lösung eines Komplexbildners behandelt wurde.

In RU-C1-21 14 849 (Institut für Technologie und Konstruktion von Katalyse- und Adsorptions-Prozessen mit Zeolith "Tseosit" SO RAN) (Derwent Abstract Nr. 2000-036595) werden Verfahren zur Herstellung von TEDA aus einem Gemisch von Monoethanolamin (MEOA), EDA und PIP an Pentasil-Zeolithen mit einem Modul (SiO₂/Al₂O₃-Molverhältnis) von 40-300 beschrieben. Die verwendeten Zeolithen werden durch Behandlung mit einem Aluminium-Chelatkomplexbildner (EDTA, Sulfosalicylsäure, TMAOH) dealuminiert. Das Patent beschreibt speziell die Umsetzung von MEOA/EDA, MEOA/PIP, EDA/PIP und EDA/MEOA/PIP-Gemischen mit NH₃ oder Wasser als Verdünnungsmittel (1:3-10) an entsprechend behandelten Zeolithen bei 350-450°C. Der Reaktoraustrag wird mittels Rektifikation in Siedebereiche getrennt. Der Bereich 160-180°C wird anschließend abgekühlt und auskristallisiert. Die Mutterlauge wird erneut als Edukt eingesetzt.

Den Verfahren nach dem Stand der Technik ist eine geringe Selektivität im Hinblick auf die Bildung von TEDA, ein sehr hoher und damit gegebenenfalls unwirtschaftlicher Anteil an Wasser als Verdünnungs- oder Lösungsmittel im Reaktorzulauf, eine unzureichende Katalysatorstandzeit, z.B. infolge von Desaktivierung, und gegebenenfalls zusätzlich eine aufwendige Katalysatorherstellung und/oder -modifizierung gemeinsam.

Der vorliegenden Erfindung lag die Aufgabe zugrunde, ein gegenüber dem Stand der Technik verbessertes wirtschaftliches Verfahren zur Herstellung von TEDA aus leicht zugänglichen Ausgangsverbindungen aufzufinden, das einfach durchzuführen ist, eine hohe Ausbeute, Selektivität und Katalysatorstandzeit und niedrigen Piperazin-Zwangsanfall aufweist und nach dem das TEDA in hoher Reinheit, Farbstabilität (d. h. kleine Farbzahl, z.B. APHA-Farbzahl nach DIN ISO 6271, die auch über längere Lagerzeiten, z.B. 6, 12 oder mehr Monate, klein bleibt) und Geruchsqualität [d.h. möglichst nur TEDA-Eigengeruch und kein Geruch nach cyclischen gesättigten 5-Ring-N-Heterocyclen oder anderen cyclischen gesättigten 6-Ring-N-Heterocyclen (z.B. PIP, N-Ethyl-piperazin) und/oder aromatischen 5- oder 6-Ring-N-Heterocyclen] anfällt.

Demgemäß wurde ein Verfahren zur Herstellung von Triethylendiamin (TEDA) durch Umsetzung von Ethylendiamin (EDA) in Gegenwart eines Zeolith-Katalysators gefunden, welches dadurch gekennzeichnet ist, dass der Zeolith-Katalysator ein oder mehrere Metalle M in der Oxidationsstufe IV als Oxide enthält, das Metall M in der Oxidationsstufe IV ausgewählt ist aus der Gruppe Ti, Zr, Ge, Hf und Sn oder Mischungen hiervon,
für M = Metall in der Oxidationsstufe IV oder M = zwei oder mehr Metalle in der Oxidationsstufe IV ein SiO2 / MO2 - Molverhältnis von größer 10 : 1 aufweist
und die Reaktionstemperatur 250 bis 500 °C beträgt

Das erfindungsgemäße Verfahren kann diskontinuierlich oder bevorzugt kontinuierlich durchgeführt werden.

Die erfindungsgemäße Umsetzung kann in der Flüssigphase oder bevorzugt in der Gasphase durchgeführt werden.

Bevorzugt wird die Umsetzung in Gegenwart eines Lösungs- oder Verdünnungsmittels durchführt.

Als Lösungs- oder Verdünnungsmittel eignen sich z.B. acyclische oder cyclische Ether mit 2 bis 12 Kohlenstoffatomen, wie Dimethylether, Diethylether, Di-n-Propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art. Weiterhin ist Ammoniak als Lösungs- oder Verdünnungsmittel geeignet.

Besonders bevorzugtes Lösungs- oder Verdünnungsmittel, insbesondere Lösungsmittel, ist Wasser.

Als Verdünnungsmittel bei Durchführung der Umsetzung in der Gasphase sind auch Inertgase wie Stickstoff (z.B. über die Sättigung des Reaktorzulaufs hinaus) oder Argon geeignet. Bevorzugt wird die Umsetzung in der Gasphase in Gegenwart von Ammoniak durchgeführt.

Beispielsweise wird die Umsetzung in Gegenwart von 2 bis 1200 Gew.-%, besonders 12 bis 1200 Gew.-%, insbesondere 14 bis 300 Gew.-%, ganz besonders 23 bis 300 Gew.-%, Lösungs- oder Verdünnungsmittel, bezogen auf eingesetztes EDA, durchgeführt.

Beispielsweise enthält das im Verfahren eingesetzte Ausgangsgemisch oder der Reaktorzulauf (= Eduktstrom bei kontinuierlicher Fahrweise) 5 bis 80 Gew.-%, besonders 10 bis 80 Gew.-%, besonders bevorzugt 20 bis 70 Gew.-%, ganz besonders bevorzugt 20 bis 65 Gew.-%, EDA und 2 bis 60 Gew.-%, besonders 10 bis 60 Gew.-%, besonders bevorzugt 15 bis 60 Gew.-%, insbesondere 20 bis 50 Gew.-%, des oder der Lösungs- und Verdünnungsmittel.

In einer besonderen Ausführungsform des erfindungsgemäßen Verfahrens werden EDA und eine oder mehrere Amin-Verbindungen, die jeweils eine 2-Aminoethyl-Gruppe, -HN-CH₂,-CH₂-, aufweisen, umgesetzt.

Solche Amin-Verbindungen sind vorzugsweise Ethanolamine (wie z.B.: Monoethanolamin (MEOA), Diethanolamin (DEOA)), Triethanolamin (TEOA), Piperazin (PIP), Diethylentriamin (DETA), Triethylentetramin (TETA), Tri(2-aminoethyl)amin, N-(2-Aminoethyl)ethanolamin (AEEA) und Piperazin-Derivate, wie z.B. N-(2-Hydroxyethyl)-piperazin (HEP) und N-(2-Aminoethyl)-piperazin (AEPIP).

PIP ist besonders bevorzugt.

Der Gehalt dieser Amin-Verbindungen im Reaktorzulauf beträgt in dieser besonderen Ausführungsform (in Summe) im allgemeinen 1 bis 1000 Gew.-%, vorzugsweise 3 bis 250 Gew.%, insbesondere 7 bis 250 Gew.-%, jeweils bezogen auf eingesetztes EDA.

Beispielsweise enthält das im Verfahren eingesetzte Ausgangsgemisch oder der Reaktorzulauf (= Eduktstrom bei kontinuierlicher Fahrweise) (in Summe) 0,5 bis 50 Gew.-%, vorzugsweise 2 bis 50 Gew.%, insbesondere 5 bis 50 Gew.-%, dieser Amin-Verbindungen.

Da auch gefunden wurde, dass in dieser besonderen Ausführungsform im Falle von eingesetztem MEOA im Ausgangsgemisch oder im Reaktorzulauf es zur Bildung von aus dem Reaktoraustrag (= Produktstrom bei kontinuierlicher Fahrweise) schwer abtrennbaren Nebenprodukten führen kann, beträgt im Fall dieser Amin-Verbindung der Gehalt im Ausgangsgemisch oder Reaktorzulauf bevorzugt 1 bis 50 Gew.-%, bezogen auf eingesetztes EDA.

Nach der Umsetzung werden die entstandenen Produkte durch übliche Verfahren, z.B. durch Destillation und/oder Rektifikation, aus dem Reaktionsaustrag isoliert; nicht umgesetzte Ausgangsstoffe können in die Umsetzung zurückgeführt werden.

So kann im Reaktionsaustrag des erfindungsgemäßen Verfahrens anfallendes PIP aus diesem, z.B. destillativ, abgetrennt und in die Umsetzung zurückgeführt werden.

Ein besonderer Vorteil des Verfahrens besteht darin, dass man bei der Aufarbeitung des Reaktionsaustrags erhaltene Zwischenfraktionen, die sowohl TEDA als auch Piperazin enthalten, und Fraktionen, die z.B. N-(2-Hydroxyethyl)-piperazin (HEP), N-(2-Aminoethyl)-piperazin (AEPIP), Diethylentriamin (DETA), Triethylentetramin (TETA), Tri(2-aminoethyl)amin und/oder N-(2-Aminoethyl)ethanolamin (AEEA) enthalten, erneut in die Umsetzung zurückführen kann.

Weiterhin können Zwangsabfälle anderer Aminverbindungen aus anderen Amincyclisierungs- / Kondensationsreaktionen der erfindungsgemäßen Umsetzung zugeführt werden, ohne dass sich die Ausbeuten an TEDA wesentlich verschlechtern.

In einer besonders bevorzugten Ausführungsform ist das erfindungsgemäße Verfahren so zu führen, dass man, insbesondere bei kontinuierlicher Durchführung (stationärer Zustand), EDA und 14 bis 300 Gew.-% Wasser und 7 bis 250 Gew.-% PIP, jeweils bezogen auf EDA,
vorzugsweise EDA und 23 bis 300 Gew.-% Wasser und 8 bis 250 Gew.-% PIP, jeweils bezogen auf EDA,
besonders bevorzugt EDA und 33 bis 250 Gew.-% Wasser und 17 bis 250 Gew.-% PIP, jeweils bezogen auf EDA,
ganz besonders bevorzugt EDA und 110 bis 185 Gew.-% Wasser und 25 bis 100 Gew.-% PIP, jeweils bezogen auf EDA,
umsetzt.

In dieser Ausführungsform kann der Anteil des PIPs oder des EDAs auch in einem Ausmaß von 0,01 bis 20 Gew.-%, beispielsweise 0,01 bis 10 Gew.-%, zugunsten des Einen und zu Lasten des Anderen erniedrigt oder erhöht werden.

Beispielsweise enthält das im Verfahren eingesetzte Ausgangsgemisch oder der Reaktorzulauf in dieser besonders bevorzugten Ausführungsform 10 bis 60 Gew.-% Wasser, 20 bis 70 Gew.-% EDA und 5 bis 50 Gew.-% PIP,
vorzugsweise 15 bis 60 Gew.-% Wasser, 20 bis 65 Gew.-% EDA und 5 bis 50 Gew.-% PIP,
besonders bevorzugt 20 bis 50 Gew.-% Wasser, 20 bis 60 Gew.-% EDA und 10 bis 50 Gew.-% PIP,
ganz besonders bevorzugt 45 bis 55 Gew.-% Wasser, 30 bis 40 Gew.-% EDA und 10 bis 30 Gew.-% PIP,
wobei der Anteil des PIPs oder des EDAs auch in einem Ausmaß wie oben beschrieben zugunsten des Einen und zu Lasten des Anderen erniedrigt oder erhöht werden kann.

In dieser besonders bevorzugten Ausführungsform des Verfahrens enthält der Reaktorzulauf neben EDA, PIP und Wasser in den oben angeführten Mengenverhältnissen oder Mengen bevorzugt weniger als 10 Gew.-%, besonders weniger als 5 Gew.-%, insbesondere weniger als 2 Gew.-%, weiterer Komponenten.

In dieser besonders bevorzugten Ausführungsform wurde gefunden, dass bei den oben angeführten Mengenverhältnissen oder Mengen der Einsatzstoffe die Reaktion, insbesondere bei kontinuierlicher Fahrweise (im stationären Zustand), so geführt werden kann, dass sich EDA fast vollständig (d.h. Umsatz größer 95 %, insbesondere größer 97 %) zu TEDA und PIP mit einer Selektivität größer 90 %, insbesondere größer 95 %, umsetzt.

Das Verfahren wird erfindungsgemäß vorzugsweise durch Einstellung eines entsprechenden EDA / PIP Verhältnisses im Reaktorzulauf (= Eduktstrom bei kontinuierlicher Fahrweise) in den o.g. Bereichen so durchgeführt, dass der Verbrauch an PIP durch Abtrennung von PIP aus dem Reaktionsaustrag und Rückführung in den Reaktorzulauf in der Gesamtbilanz gegen Null geht (z.B. 0 bis 30 kg, insbesondere 0 bis 15 kg, ganz besonders 0 bis 10 kg, pro 100 kg TEDA im Reaktionsaustrag), insbesondere Null ist, und gleichzeitig das eingesetzte EDA vollständig (> 95 %, insbesondere > 97 %, ganz besonders > 99 %) umgesetzt wird. D.h. im Ergebnis während der kontinuierlichen Fahrweise im wesentlichen kein zusätzliches PIP dem erfindungsgemäßen Verfahren zugeführt wird.

Da bei einer solchen Reaktionsführung erfindungsgemäß die Menge an ausgetragenem EDA gegen Null geht, ist die Auftrennung des Reaktoraustrages, z.B. durch Destillation und/oder Rektifikation, nach dieser Verfahrensvariante besonders einfach.

Die Reaktionstemperatur im erfindungsgemäßen Verfahren beträgt bevorzugt 300 bis 400°C, besonders bevorzugt 310 bis 390°C.

Die Eduktkomponenten oder der Reaktorzulauf werden vorteilhafterweise vortemperiert.

Für die Durchführung des erfindungsgemäßen Verfahrens haben sich weiterhin folgende Reaktionsbedingungen als günstig erwiesen:
- eine WHSV (weight hourly space velocity) bezogen auf in die Umsetzung eingesetzte Amine von 0,05 bis 6 h⁻¹, vorzugsweise von 0,1 bis 1 h⁻¹, besonders bevorzugt von 0,3 bis 1 h⁻¹, und
- ein Druck (absolut) von 0,01 bis 40 bar, besonders 0,1 bis 10 bar, vorzugsweise von 0,8 bis 2 bar.

Als Reaktoren, in denen das erfindungsgemäße Verfahren durchgeführt wird, eignen sich Rührbehälter, insbesondere Rohrreaktoren und Rohrbündelreaktoren.

Der Zeolith-Katalysator ist im Reaktor bevorzugt als Festbett angeordnet.

Die Umsetzung in der Flüssigphase kann z.B. in der Suspensions-, Riesel- oder Sumpffahrweise erfolgen.

Die bevorzugte Umsetzung in der Gasphase kann in einem Katalysator-Wirbelbett oder bevorzugt -Festbett erfolgen.

Im Folgenden Absatz wird zusätzlich beispielhaft geschildert, wie das erfindungsgemäße Verfahren durchgeführt werden kann:
Der Reaktorzulauf (Zusammensetzung: wie oben beschrieben) wird in einem Verdampfer, der gegebenenfalls Bestandteil des eigentlichen Reaktors sein kann, bei einer Temperatur von 250-500°C in die Gasphase überführt und auf den Katalysator geleitet. Der am Reaktorausgang gasförmig anfallende Reaktionsaustrag wird durch im Kreislauf gepumpten verflüssigten Reaktionsaustrag bei Temperaturen von 20-100°C, bevorzugt bei 80°C gequencht. Dieser verflüssigte Reaktionsaustrag wird wie folgt aufgearbeitet: In einer ersten Destillationsstufe werden Leichtsieder wie Acetaldehyd, Ethylamin, Ammoniak und Wasser sowie heterocyclische Verbindungen, die als Nebenkomponenten in der Synthese gebildet werden, abgetrennt. In einer zweiten Destillationsstufe wird der Reaktionsaustrag von Piperazin befreit, welches erneut dem Reaktorzulauf zugeführt wird. Der Strom des abgetrennten Piperazins kann dabei bis zu 20 Gew-% TEDA enthalten. (Alternativ ist auch die gleichzeitige Abtrennung von Wasser und Piperazin möglich, die gemeinsam in den Reaktorzulauf zurückgeführt werden können). In einer dritten Destillationsstufe wird das Wertprodukt TEDA destillativ aus dem Reaktionsaustrag gewonnen und bei Bedarf, z.B. in einer nachfolgenden Kristallisationsstufe (z.B. wie weiter unten beschrieben), weiter aufgearbeitet.

Mit dem erfindungsgemäßen Verfahren werden unter anderem folgende Vorteile erzielt:
- Das Verfahren erlaubt das als Edukt eingesetzte EDA je nach Preis und Verfügbarkeit durch eine oder mehrere Amin-Verbindungen, die jeweils eine 2-Aminoethyl-Gruppe, -HN-CH₂,-CH₂,-, aufweisen (siehe oben), zu ersetzen oder diese Amin-Verbindungen zusätzlich dem Reaktorzulauf zuzufügen.
- Das im wesentlichen einzige Nebenprodukt Piperazin kann bei geeigneter Reaktionsführung wie oben beschrieben in den Prozess erneut eingeschleust und dabei zu TEDA umgesetzt werden. Auch Gemische aus nicht umgesetztem Piperazin und TEDA können dem Katalysator erneut zugeführt werden, da sich gezeigt hat, dass TEDA unter den Reaktionsbedingungen stabil ist.
- Bei geeigneter Wahl des EDA/PIP-Verhältnisses im Reaktorzulauf wie oben beschrieben geht der Verbrauch an PIP in der Gesamtbilanz gegen Null, weil bei Rückführung des im Reaktionsaustrag enthaltenen PIPs ein mengenmässig konstanter PIP-Strom im Reaktorzulauf erhalten wird und somit in der Gesamtbilanz als einziges Amin ausschliesslich EDA der Reaktion kontinuierlich von aussen zugeführt werden muss.
- Es wird eine hohe Selektivität und ein hoher Umsatz, bezogen auf die Umsetzung von EDA zu TEDA, erzielt.
- Aufgrund der erfindungsgemäß verwendeten Zeolith-Katalysatoren werden bei der Umsetzung von EDA und gegebenenfalls der o.g. Amin-Verbindungen weniger Nebenprodukte gebildet, was zu einer vereinfachten Aufarbeitung des verfahrensgemäß anfallenden TEDAs zur Erreichung der geforderten Produktspezifikationen (Reinheit, Farbzahl, Geruch) führt.

Der Zeolith, der als Katalysator in dem erfindungsgemäßen Verfahren zur Herstellung von TEDA eingesetzt wird, weist eine Skellet-Struktur, die hauptsächlich aus Siliciumdioxid (SiO₂) besteht, auf.

Neben Siliciumdioxid kann der Zeolith noch Spuren von anderen 4-wertigen Metallen M (Oxidationsstufe der Metalle IV) in Form von Metalloxiden M_{2/z}O (z = 4) enthalten, wobei das molare Verhältnis von Siliciumdioxid und dem Metalloxid, SiO₂ / M_{2/z}O (dieses molare Verhältnis wird auch ,Modul' genannt), für 4-wertige Metalle (z = 4) größer 10, ist.

Die im erfindungsgemäßen Verfahren bevorzugt verwendeten Zeolith-Katalysatoren weisen für M = Metall in der Oxidationsstufe IV oder M = zwei oder mehr Metalle in der Oxidationsstufe IV ein SiO₂, / MO₂ - Molverhältnis von größer 10 bis 40000 : 1, insbesondere von größer 25 bis 5000 : 1, auf.

Der im erfindungsgemäßen Verfahren verwendete Zeolith-Katalysator ist bevorzugt vom Pentasil-Typ.

Die obere Grenze des Moduls (40000) ist nur durch die Reinheit der Ausgangsubstanzen (Restspuren an M bzw. Verbindungen von M) und die Reinheit und chemische Beständigkeit der bei der Synthese des Zeoliths eingesetzten Apparate begrenzt.

Bei einem Modul unterhalb der angegebenen Grenze nimmt die Brönsted- und Lewis-Aciditätsdichte (Aciditätsdichte: saure Zentren/Katalysatorgesamtoberfläche) der Zeolithe deutlich zu, die erreichbare TEDA-Ausbeute und -Selektivität und die Katalysatorstandzeit deutlich ab und der Aufwand zur Aufreinigung des TEDAs deutlich zu.

Überraschenderweise wurde gefunden, dass Zeolithe mit vierwertigen Metallen in Form von Metalloxiden (M für z = 4) im Zeolithgitter erfindungsgemäß eine verbesserte Selektivität bezüglich TEDA erzielen.

Für den Zeolith-Katalysator, bevorzugt vom Pentasil-Typ, mit Modulen wie oben angegeben bestehen weder zusätzliche Erfordernisse bezüglich des Zeolith-Materials als solchem noch bezüglich des Verfahrens nach dem dieses erhältlich ist.

Bei dem im erfindungsgemäßen Verfahren verwendeten Zeolith-Katalysator, der neben SiO₂, ein oder mehrere Metalle M in den Oxidationsstufen IV als Oxide enthält, ist das Metall M in der Oxidationsstufe IV erfindungsgemäß ausgewählt aus der Gruppe Ti, Zr, Ge, Hf und Sn oder Mischungen hiervon.

Bevorzugt sind Zeolithe, bei denen M für Titan, Zirkonium oder Germanium steht. Solche, bei denen M für Titan steht, sind besonders bevorzugt.

Als erfindungsgemäß einzusetzende Zeolith-Katalysatoren des Pentasil-Typs sind z.B. folgende Typen geeignet: ZSM-5 (wie z.B. in US-A-3,702,886 offenbart), ZSM-11 (wie z.B. in US-A-3,709,979 offenbart), ZSM-23, ZSM-53, NU-87, ZSM-35, ZSM-48 und Mischstrukturen aus mindestens zwei der oben genannten Zeolithe, insbesondere ZSM-5 und ZSM-11, sowie deren Mischstrukturen.

Besonders bevorzugt sind für das erfindungsgemäße Verfahren Zeolithe mit MFI-, MEL-Sruktur, MEL/MFI- oder MFI/MEL-Mischstruktur.

Die erfindungsgemäß verwendeten Zeolithe sind kristalline Metallsilikate mit geordneter Kanal- und Käfigstruktur, die Mikroporen aufweisen. Das Netzwerk solcher Zeolithe ist aufgebaut aus SiO₄-und M_{2/z}O (z = 4) - Tetraedern, die über gemeinsame Sauerstoffbrücken verbunden sind. Eine Übersicht über die bekannten Strukturen findet sich beispielsweise bei W.M. Meier, D.H. Olsen und Ch. Baerlocher in "Atlas of Zeolite Structure Types", Elsevier, 4. Auflage, London 1996.

Ferner sind erfindungsgemäß Zeolithe einsetzbar, die kein Aluminium (M = Al) enthalten und bei denen im Zeolithgitter das Si(IV) teilweise durch ein Metall M(IV), wie z.B. Ti, Zr, Ge, Hf und/oder Sn ersetzt ist.

(IV = Oxidationsstufe 4).

Überlicherweise stellt man die genannten Zeolithe dadurch her, dass man eine Mischung aus einer SiO₂-Quelle sowie aus einer Metall-Quelle (z.B. M = Ti, Zr, Ge, Hf und/oder Sn in den Oxidationsstufen wie oben beschrieben) und einer stickstoffhaltigen Base als Templat ("Schablonen-Verbindung"), wie z.B. Tetraalkylammoniumsalz, gegebenenfalls noch unter Hinzufügen basischer Verbindungen (z.B. Laugen), in einem Druckbehälter unter erhöhter Temperatur im Zeitraum mehrerer Stunden oder einiger Tage umsetzt, wobei ein kristallines Produkt entsteht. Dieses wird abgetrennt (z.B. abfiltriert, sprühgetrocknet oder ausgefällt), gewaschen, getrocknet und zur Entfernung der organischen Stickstoffbase bei erhöhter Temperatur kalziniert (siehe unten). Wahlweise ist auch die Synthese ohne Templat möglich, sofern die Bildung des Zeolithen gewährleistet ist. In dem so erhaltenen Pulver liegt das Metall (z.B. M = Ti, Zr, Ge, Hf und/oder Sn in den Oxidationsstufen wie oben beschrieben) zumindest teilweise innerhalb des Zeolithgitters in wechselndem Anteil mit 4-, 5- oder 6-facher Koordination vor.

Die erfindungsgemäß verwendeten Zeolithe sind über das beschriebene Verfahren herstellbar und/oder kommerziell im Handel erhältlich.

Liegt der erfindungsgemäß einzusetzende Zeolith-Katalysator, bevorzugt des Pentasil-Typs, aufgrund der Art der Produktion nicht zumindest teilweise in der bevorzugten aciden H⁺-Form und/oder NH₄⁺-Form vor, sondern z.B. in der Na⁺-Form (oder einer anderen beliebigen Metallsalzform), so kann dieser, gemäß dem Stand der Technik, durch lonenaustausch, z.B. mit Ammoniumionen, und anschließender Kalzinierung (s. unten) zumindest partiell in die bevorzugte H⁺- und/oder NH₄⁺-Form überführt werden. Die ebenso literaturbekannte, Behandlung mit verdünnter Protonen-Säure, z.B. Mineralsäure, zur Überführung des Zeolithen zumindest teilweise in die H⁺-Form ist genauso praktikabel. Geeignet sind hier alle Protonen-Säuren, wie z.B. Salzsäure oder Schwefelsäure (s. unten).

Anschließend ist es möglich, den so ausgetauschten Zeolith-Katalysator durch Ionenaustausch mit einer entsprechenden Metallsalzlösung (Metall Me = Alkalimetall, Erdalkalimetall, Übergangsmetall) in eine gewünschte Me⁺-Form zu überführen, die noch H⁺ und/oder NH₄⁺ enthält.

Um eine möglichst hohe Selektivität, hohe Umsätze sowie besonders lange Katalysator-Standzeiten zu erreichen, kann es vorteilhaft sein, die anspruchsgemäßen Zeolith-Katalysatoren zu modifizieren.

Eine geeignete Modifizierung der Zeolith-Katalysatoren besteht darin, wie in ,J. Weitkamp et al., Catalysis and Zeolites, Kap. 3: Modification of Zeolites, Springer Verlag, 1999' beschrieben, dass man das zeolithische Material - verformt oder unverformt - einer Behandlung, gemäß bekanntem Stand der Technik (EP-A-382 055, S. 4, Zeile 2ff + Zeile 20ff; DE-C2-24 34 913, S. 3 Zeile 23ff; US-A-5,041,548, S. 4, Zeile 27ff), mit konzentrierten oder verdünnten Protonen-Säuren - wie z.B. Salzsäure, Schwefelsäure, Flusssäure, Phosphorsäure, einer Carbonsäure, Dicarbonsäure oder Polycarbonsäure - und/oder Komplexbildnern - wie z.B. Acetylacetonat (acac), Nitrilotriessigsäure, Sulfosalicylsäure, Ethylendiaminotetraessigsäure (EDTA) -, z.B. gemäß EP-A-842 936 und RU-C1-21 14 849, und/oder Wasserdampf unterwirft.

In einer besonderen Ausführungsform kann eine Dotierung der im erfindungsgemäßen Verfahren verwendeten Zeolithe durch Auftragen von Übergangsmetallen der I. bis VIII. Nebengruppe, bevorzugt die der I., II., IV. und VIII. Nebengruppe, besonders bevorzugt Zn, Ti, Zr, Fe, Co, Ni, Cr, V, auf diese erfolgen.

Die Auftragung kann durch Tränken des im erfindungsgemäßen Verfahren verwendeten Zeoliths in wässrigen Metallsalzlösungen, durch Aufsprühen entsprechender Metallsalzlösungen auf den Zeolith oder durch andere geeignete, im Stand der Technik bekannte Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitrokomplexe oder Aminkomplexe der entsprechenden Metalle, wobei die Nitrate und Nitrosylnitrate bevorzugt sind. Bei Zeolithen, die mit mehreren Metallen dotiert sind, können die Metallsalze- bzw. Metallsalzlösungen gleichzeitig oder nacheinander aufgebracht werden.

Die mit den Metallsalzlösungen beschichteten oder getränkten, Zeolithe werden anschließend, vorzugsweise bei Temperaturen zwischen 60 und 150°C, getrocknet und wahlweise bei Temperaturen zwischen 200 und 950°C, vorzugsweise zwischen 400 und 750°C, kalziniert. Bei getrennter Auftränkung wird der Katalysator nach jedem Tränkschritt getrocknet und wahlweise, wie oben beschrieben, kalziniert. Die Reihenfolge in der die Übergangsmetalle aufgetränkt werden, ist dabei frei wählbar. Wahlweise werden anschließend die beschichteten und getrockneten sowie wahlweise kalzinierten Zeolithe durch Behandlung in einem Gasstrom, der freien Wasserstoff enthält, bei Temperaturen zwischen 30 und ungefähr 600°C, vorzugsweise zwischen 150 und ungefähr 450°C, aktiviert. Vorzugsweise besteht der Gasstrom aus 50 bis 100 Vol.-% Wasserstoff und 0 bis 50 Vol.-% Stickstoff.

Die Übergangsmetalllösungen werden in einer solchen Menge auf den Zeolith aufgebracht, dass der Gesamtgehalt an Übergangsmetall, jeweils bezogen auf das Gesamtgewicht des Katalysators, ungefähr 0,01 bis ungefähr 10 Gew.-%, vorzugsweise ungefähr 0,01 bis 5 Gew.-%, weiter bevorzugt ungefähr 0,01 bis ungefähr 2 Gew.-% und inbesondere ungefähr 0,05 bis 1 Gew.-% beträgt.

Die Übergangsmetalloberfläche auf dem Katalysator beträgt dabei insgesamt vorzugsweise ungefähr 0,01 bis ungefähr 10 m²/g, weiter bevorzugt 0,05 bis 5 m²/g und inbesondere ungefähr 0,05 bis 3 m²/g (m² pro g des Katalysators). Die Metalloberfläche wird mittels der von J. LeMaitre et al. In "Characterization of Heterogeneous Catalysts", Hrsg. Francis Delanny, Marcel Dekker, New York 1984, S. 310 - 324, beschriebenen Chemisorptionsverfahren bestimmt.

Zur Erhöhung der Standfestigkeit können die erfindungsgemäß einzusetzenden Zeolithe geträgert werden, z.B. auf Cellulosematerialien, Tonen, Polymere, Metalle, Graphite, Bindemitteln oder Metalloxiden wie Tonerden, Aluminiumoxid, Siliciumdioxid. Weiterhin ist es möglich dieses als Granulat, in Kugelform oder auf Glas- oder andere Körper wie z.B. Geweben (insbesondere Metallgeweben) jeglicher Art aufgebracht einzusetzen.

Als verfestigende Formgebungsprozesse für die erfindungsgemäß einzusetzenden Zeolithe können im Prinzip alle Methoden zur Erlangung einer entsprechenden Formung verwendet werden. Bevorzugt werden Verfahren, bei denen die Formgebung durch Tablettierung oder Extrusion erfolgt. Besonders bevorzugt werden Verfahren, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion bzw. der Tablettierung zweckmäßigerweise ein Mischungs- oder Knetprozess vorgeschaltet. Gegebenenfalls erfolgt nach der Extrusion/Tablettierung noch ein Kalzinierungsschritt. Die erhaltenen Formkörper werden gewünschtensfalls zerkleinert, vorzugsweise zu Granulat oder Splitt mit einem Partikeldurchmesser von 0,5 bis 5 mm, inbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der geformte, erfindungsgemäß einzusetzende Zeolith bis zu 80 Gew.-% Bindemittel, bezogen auf die Gesamtmasse des Katalysators. Besonders bevorzugte Bindemittelgehalte sind 1 bis 60 Gew.-%, insbesondere 20 bis 45 Gew.-%. Als Bindemittel eignen sich im Prinzip alle für derartige Zwecke eingesetzte Verbindungen, bevorzugt werden Verbindungen, inbesondere Oxide, des Siliciums, Aluminiums, Bors, Phosphors, Zirkoniums und/oder Titans. Von besonderem Interesse als Bindemittel ist Siliciumdioxid, wobei das SiO₂, auch als Kieselsol oder in Form von Tetraalkoxysilanen in den Formgebungsprozess eingebracht werden kann. Auch als Bindemittel verwendbar sind Oxide des Magnesiums und Berylliums sowie Tone, z.B. Montmorillonit, Kaoline, Bentonite, Halloysite, Dickite, Nacrite und Anauxite.

Als Hilfsmittel für die verfestigenden Formgebungsprozesse sind beispielsweise Verstrangungshilfsmittel für die Extrusion zu nennen, ein übliches Verstrangungsmittel ist Methylcellulose. Derartige Mittel werden in der Regel in einem nachfolgendem Kalzinierungsschritt vollständig verbrannt.

Die Kalzinierung des erfindungsgemäß einzusetzenden Zeolith-Katalysators erfolgt bei Temperaturen von 250 bis 950°C, bevorzugt bei 400 bis 750°C, besonders bevorzugt bei 450 bis 600°C, für die Dauer von im allgemeinen mindestens einer Stunde, bevorzugt für 2 - 5 Stunden. Die Kalzinierung erfolgt in einer Gasatmosphäre, z.B. Stickstoff-, Luft-, Edelgas-Atmospäre. In der Regel wird in sauerstoffhaltiger Atmosphäre kalziniert, wobei der Sauerstoffgehalt 0,1 bis 90 Vol.-%, bevorzugt 0,2 bis 22 Vol.-%, besonders bevorzugt 10 bis 22 Vol.-%, beträgt. Die Verwendung von anderen Sauerstoff-liefernden Substanzen ist ebenfalls möglich. Der obige Begriff "Sauerstoff-liefernde Substanzen" umfasst alle Substanzen, die in der Lage sind, unter den angegebenen Kalzinierbedingungen Sauerstoff abzugeben. Insbesondere zu nennen sind: Stickoxide der Formel NₓO_{y}, wobei x und y so gewählt werden, dass sich ein neutrales Stickoxid ergibt, N₂O, N₂O haltiger Abgasstrom aus einer Adipinsäureanlage, NO, NO₂, Ozon oder ein Gemisch aus zwei oder mehr davon. Bei Verwendung von CO₂ als Sauerstoff-liefernde Substanz werden bevorzugt Temperaturen von 500°C bis 800°C während der Kalzination eingestellt. Eine Kalzinierung unter Wasserdampfatmosphäre ist ebenfalls möglich.

Erfindungsgemäß wurde weiterhin erkannt, dass nach dem Einsatz des erfindungsgemäß verwendeten Zeolith-Katalysators, dieser unabhängig von seiner Form, z.B. nach Abnahme der Aktivität und/ oder der Selektivität, durch ein Verfahren regeneriert werden kann, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Ein solches Regenerierungsverfahren ist unter anderem in der WO 98/55228 und der DE-A1-19 72 39 49 beschrieben, deren Offenbarung durch diesbezügliche Bezugnahme hiermit vollumfänglich auch zum Gegenstand der vorliegenden Anmeldung gemacht wird.

Nach der Regeneration ist die Aktivität und/oder die Selektivität des Katalysators, verglichen mit dem Zustand unmittelbar vor der Regeneration, erhöht.

Der zu regenerierende, erfindungsgemäß einzusetzende Zeolith-Katalysator wird entweder in der Umsetzungsvorrichtung (Reaktor) oder in einem externen Ofen in einer Atmosphäre, die 0,1 bis ungefähr 20 Volumen-Anteile von Sauerstoff-liefernden Substanzen, besonders bevorzugt 0,1 bis ungefähr 20 Volumen-Anteile Sauerstoff, enthält, auf eine Temperatur im Bereich von ungefähr 250°C bis 800°C, vorzugsweise ungefähr 400°C bis 550°C und insbesondere ungefähr 450°C bis 500°C, aufgeheizt. Dabei wird das Aufheizen vorzugsweise mit einer Aufheizrate von ungefähr 0,1 °C/Min. bis ungefähr 20°C/Min., vorzugsweise ungefähr 0,3°C/Min bis ungefähr 15°C/Min. und insbesondere 0,5°C/Min. bis 10°C/Min., durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators.

Sinkt die Temperatur des Abgasstroms am Reaktorausgang trotz steigender Mengen an Sauerstoff-liefernden Substanzen im Gasstrom, so ist das Abbrennen der organischen Beläge beendet. Die Dauer der Behandlung beträgt im allgemeinen jeweils ungefähr 1 bis 30, vorzugsweise ungefähr 2 bis ungefähr 20 und insbesondere ungefähr 3 bis ungefähr 10 Stunden.

Beim anschließenden Abkühlen des so regenerierten Katalysators ist darauf zu achten, dass das Abkühlen nicht zu schnell erfolgt ("Abschrecken"), da sonst die mechanische Festigkeit des Katalysators negativ beeinflusst werden kann.

Es kann erforderlich sein, den Katalysator nach der durchgeführten Regeneration durch Kalzination, wie oben beschrieben, einer Spülung mit Wasser und/oder verdünnten Säuren, wie z.B. Salzsäure, zu unterziehen, um evtl. die durch Verunreinigung der Edukte verbleibende anorganische Beladung des Katalysators (Alkalispuren etc.) zu entfernen. Anschließend kann eine erneute Trocknung und/oder Kalzination des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellverfahrens kann darin bestehen, dass das erfindungsgemäße, kontinuierliche Verfahren zur Synthese von TEDA bei der Regeneration des erfindungsgemäßen Katalysators nicht unterbrochen werden muss, um den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung von EDA zur Verfügung steht.

Das erfindungsgemäß erhaltene TEDA kann zur Verbesserung seiner Reinheit aus geeigneten Lösungsmitteln (z.B. Pentan, Hexan) umkristallisiert werden. Meist ist dies jedoch nicht erforderlich, da TEDA nach dem erfindungsgemäßen Verfahren mit Reinheiten größer 95 Gew.-%, z.B. größer 97 Gew.-%, hergestellt werden kann.

In einer besonderen Ausgestaltung ist das anspruchsgemäße TEDA-Herstellverfahren kombiniert mit dem sich anschließenden TEDA-Verfahren gemäß der älteren EP-Anmeldung Nr. 00114475.7 vom 06.07.00 (BASF AG).

Gemäß dieser Kombination wird zunächst TEDA anspruchsgemäß hergestellt. Bei der sich anschließenden Aufarbeitung des TEDAs (z.B. destillativ), die mehrstufig sein kann, wird das TEDA, bevorzugt in der letzten Aufarbeitungsstufe (insbesondere Destillations- bzw. Rektifikationsstufe), verdampft und das, z.B. am Kopf oder in einem Seitenabzug der Destillationskolonne erhaltene, dampfförmige TEDA, das bevorzugt eine Reinheit von größer 95 Gew.-%, insbesondere von größer 97 Gew.-% besitzt, in ein flüssiges Lösungsmittel einleitet. Diese Einleitung des dampfförmigen TEDAs direkt in ein flüssiges Lösungsmittel wird im Folgenden auch, TEDA-Quench' genannt.

Durch anschließende Auskristallisation des TEDAs aus der so erhaltenen Lösung wird reines TEDA mit hoher Qualität erhalten.

Das flüssige Lösungsmittel wird im allgemeinen aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt.

Zur Herstellung einer Lösung von reinem TEDA gemäß obiger Verfahrenskombination, die z.B. als Katalysatorlösung bei der Polyurethanschaumherstellung verwendet werden kann, wird als Lösungsmittel für den TEDA-Quench bevorzugt ein Alkohol (z.B. Ethylenglykol, 1,4-Butandiol, bevorzugt Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA, ganz besonders kleiner 50 APHA.

Zur Herstellung von reinem (kristallinem) TEDA gemäß obiger Verfahrenskombination wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z.B. Hexan, Heptan, bevorzugt Pentan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im allgemeinen mindestens 99,5 Gew.-%, insbesondere mindestens 99,8 Gew.-%. Gehalt an PIP kleiner 0,1 Gew.-%, insbesondere kleiner 0,05 Gew.-%, Gehalt an N-Ethylpiperazin kleiner 0,02 Gew.-%, insbesondere kleiner 0,01 Gew.-%) und die Farbzahl einer 33 Gew.-%igen Lösung in Dipropylenglykol beträgt kleiner 50 APHA, insbesondere kleiner 30 APHA.

(Alle APHA-Zahlen nach DIN ISO 6271).

Die Einleitung des dampfförmigen TEDAs in das flüssige Lösungsmittel erfolgt in einem Quenchapparat, z.B. bevorzugt in einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Im allgemeinen wird die Temperatur im TEDA-Quench durch Temperierung des eingesetzten Lösungsmittels und/oder des Quenchapparates auf 20 bis 100°C, bevorzugt 30 bis 60°C, eingestellt. Der Absolutdruck im TEDA-Quench beträgt im allgemeinen 0,5 bis 1,5 bar.

Im allgemeinen wird so verfahren, dass, je nach Art des Lösungsmittels, beim TEDA-Quench zunächst Lösungen mit einem TEDA-Gehalt von ca. 1 bis 50 Gew.-%, bevorzugt 20 bis 40 Gew.-%, erhalten werden.

### Beispiele

### Katalysator A:

Zunächst erfolgte die Herstellung eines TS-1 Zeolith-Pulvers (Pentasil, MFI-Struktur) gemäß Beispiel 1 von DE-A-196 23 611 (SiO₂/TiO₂ = 40 [mol/mol]). Das Zeolith-Pulver wurde vor dem Verstrangen mit 20 Gew.-% SiO₂, (bezogen auf die Gesamtmasse der fertigen Stränge) mit 5M HCI behandelt, mehrmals mit Wasser ausgewaschen und bei 500°C für 5 h kalziniert. Die 2 mm-Stränge wurden abschließend bei 500°C für 5 h kalziniert.

### Beispiel 1: Kontinuierliche Herstellung von TEDA

Der Katalysator gemäß untenstehender Tabelle wurde in einer Gasphasenapparatur eingesetzt (beheizter Rohrreaktor: Länge: 1000 mm, Durchmesser 6 mm). Eduktgemisch: 35 % EDA, 15 % PIP, 50.0 % Wasser (alle Angaben in Gew-%). Das wässrige Eduktgemisch wurde direkt in den Reaktor gepumpt und im oberen Teil bei einer Reaktionstemperatur von 345°C verdampft bevor es drucklos auf den Katalysator geleitet wurde. Belastung: 1.0 kg Eduktgemisch/kg Katalysator*h. Die Reaktionsprodukte wurden am Reaktorausgang in einem Kühler kondensiert, gesammelt und ein aliquoter Anteil gaschromatographisch analysiert.

### GC-Analytik:

Säule: RTX-5, 30 m; Temperaturprogramm: 80°C - 5°C/Min. - 280°C, Detektor: FID, interner Standard: N-Methylpyrrolidon (NMP).

### Auswertung:

Tab. 1: Synthese von Triethylendiamin (TEDA) aus Ethylendiamin (EDA) und Piperazin (PIP); Feed: 35:15:50 EDA:PIP:H₂O in Gew.-%; Modul: SiO₂/TiO₂-Molverhältnis der verwendeten Zeolithen.

| Beispiel | Katalysator | Modul [SiO₂/MₓOₓ₊₁] | U_{EDA} [%] | U_{PIP} [%] | S_{TEDA} [%] |
|---|---|---|---|---|---|
| 1 | A | 40 (M=Ti,x=1) | >95 | 11 | 95 |

| | | | | | |
|---|---|---|---|---|---|
| (U = Umsatz im Gew.-% bezogen auf die eingesetzte Menge des in der Tabelle genannten Stoffs (EDA bzw. PIP); S = Selektivität, bezogen auf umgesetzte -CH₂-CH₂- -Einheiten stammend aus EDA und PIP). | | | | | |

## Patentansprüche

1. Verfahren zur Herstellung von Triethylendiamin (TEDA) durch Umsetzung von Ethylendiamin (EDA) in Gegenwart eines Zeolith-Katalysators, **dadurch gekennzeichnet, dass** der Zeolith-Katalysator ein oder mehrere Metalle M in der Oxidationsstufe IV als Oxide enthält, das Metall M in der Oxidationsstufe IV ausgewählt ist aus der Gruppe Ti, Zr, Ge, Hf und Sn oder Mischungen hiervon,
für M = Metall in der Oxidationsstufe IV oder M = zwei oder mehr Metalle in der Oxidationsstufe IV ein SiO₂, / MO₂ - Molverhältnis von größer 10 : 1 aufweist
und die Reaktionstemperatur 250 bis 500°C beträgt.

2. Verfahren nach Anspruch 1, **dadurch gekennzeichnet, dass** man die Umsetzung kontinuierlich durchführt.

3. Verfahren nach den Ansprüchen 1 oder 2, **dadurch gekennzeichnet, dass** man die Umsetzung in der Gasphase durchführt.

4. Verfahren nach einem der vorhergehenden Ansprüche, **dadurch gekennzeichnet, dass** man die Umsetzung in Gegenwart eines Lösungs- oder Verdünnungsmittels durchführt.

5. Verfahren nach einem der vorhergehenden Ansprüche, **dadurch gekennzeichnet, dass** man die Umsetzung in Gegenwart von Wasser und/oder Ammoniak durchführt.

6. Verfahren nach einem der vorhergehenden Ansprüche, **dadurch gekennzeichnet, dass** man die Umsetzung in Gegenwart von 2 bis 1200 Gew.-% Wasser, bezogen auf eingesetztes EDA, durchführt.

7. Verfahren nach einem der vorhergehenden Ansprüche, **dadurch gekennzeichnet, dass** man die Umsetzung in Gegenwart von 14 bis 300 Gew.-% Wasser, bezogen auf eingesetztes EDA, durchführt.

8. Verfahren nach einem der vorhergehenden Ansprüche, **dadurch gekennzeichnet, dass** man EDA und eine oder mehrere der Amin-Verbindungen aus der Gruppe Monoethanolamin, Diethanolamin, Triethanolamin, Piperazin (PIP), Diethylentriamin, Triethylentetramin, Tri(2-aminoethyl)amin, N-(2-Aminoethyl)ethanolamin, N-(2-Hydroxyethyl)-piperazin und N-(2-Aminoethyl)-piperazin umsetzt.

9. Verfahren nach einem der vorhergehenden Ansprüche, **dadurch gekennzeichnet, dass** man EDA und 1 bis 1000 Gew.-% Piperazin (PIP), bezogen auf EDA, umsetzt.

10. Verfahren nach einem der Ansprüche 1 bis 8, **dadurch gekennzeichnet, dass** man EDA und 7 bis 250 Gew.-% Piperazin (PIP), bezogen auf EDA, umsetzt.

11. Verfahren nach einem der Ansprüche 1 bis 8, **dadurch gekennzeichnet, dass** man EDA, 8 bis 250 Gew.-% PIP und 23 bis 300 Gew.-% Wasser, jeweils bezogen auf EDA, umsetzt.

12. Verfahren nach einem der vorhergehenden Ansprüche, **dadurch gekennzeichnet, dass** man nach der Umsetzung anfallendes PIP abtrennt und in die Umsetzung mit EDA zurückführt.

13. Verfahren nach dem vorhergehenden Anspruch, **dadurch gekennzeichnet, dass** der Verbrauch an PIP in der Gesamtbilanz 0 bis 30 kg pro 100 kg TEDA beträgt.

14. Verfahren nach einem der vorhergehenden Ansprüche, **dadurch gekennzeichnet, dass** die Reaktionstemperatur 310 bis 390 °C beträgt.

15. Verfahren nach einem der vorhergehenden Ansprüche, **dadurch gekennzeichnet, dass** der Absolutdruck 0,1 bis 10 bar beträgt.

16. Verfahren nach einem der vorhergehenden Ansprüche, **dadurch gekennzeichnet, dass** die WHSV (weight hourly space velocity) bezogen auf in die Umsetzung eingesetzte Amine 0,05 bis 6 h⁻¹ beträgt.

17. Verfahren nach einem der vorhergehenden Ansprüche, **dadurch gekennzeichnet, dass** der Zeolith-Katalysator für M = Metall in der Oxidationsstufe IV oder M = zwei oder mehr Metalle in der Oxidationsstufe IV ein SiO₂ / MO₂ - Molverhältnis von größer 10 bis 40000 : 1 aufweist.

18. Verfahren nach einem der vorhergehenden Ansprüche, **dadurch gekennzeichnet, dass** der Zeolith-Katalysator vom Pentasil-Typ ist.

19. Verfahren nach einem der vorhergehenden Ansprüche, **dadurch gekennzeichnet, dass** der Zeolith-Katalysator vom Struktur-Typ MFI, MEL oder deren Mischstrukturen (MEL/MFI, MFI/MEL) ist.

20. Verfahren nach einem der vorhergehenden Ansprüche, **dadurch gekennzeichnet, dass** der Zeolith-Katalysator vor dem Einsatz in dem Verfahren mit einer Protonen-Säure behandelt wird.

21. Verfahren nach einem der vorhergehenden Ansprüche, **dadurch gekennzeichnet, dass** der Zeolith-Katalysator mit einem oder mehreren Übergangsmetallen dotiert ist.

22. Verfahren nach einem der vorhergehenden Ansprüche, **dadurch gekennzeichnet, dass** der Zeolith-Katalysator als Bindemittel Siliciumdioxid enthält.

23. Verfahren nach einem der vorhergehenden Ansprüche, **dadurch gekennzeichnet, dass** zumindest teilweise ein Zeolith-Katalysator eingesetzt wird, der in einer Gasatmosphäre in Gegenwart von Sauerstoff oder Sauerstoff-liefernden Substanzen bei einer Temperatur im Bereich von 250 bis 800 °C behandelt oder regeneriert wurde.

24. Verfahren nach einem der vorhergehenden Ansprüche, **dadurch gekennzeichnet, dass** das Verfahren ohne Unterbrechung in mindestens zwei parallel geschaltenen Reaktoren durchgeführt wird, von denen jeweils einer zur Regeneration des Zeolith-Katalysators vom Edukt- und Produktstrom abkoppelbar ist.

25. Verfahren zur Herstellung einer Lösung von TEDA, **dadurch gekennzeichnet, dass** man TEDA nach einem der vorhergehenden Ansprüche herstellt, das hergestellte TEDA verdampft und das dampfförmige TEDA in ein flüssiges Lösungsmittel einleitet.

26. Verfahren zur Herstellung von TEDA, **dadurch gekennzeichnet, dass** man eine Lösung von reinem TEDA gemäß dem vorhergehenden Anspruch herstellt und anschließend das TEDA aus dieser Lösung auskristallisiert.

27. Verfahren nach einem der beiden vorhergehenden Ansprüche, **dadurch gekennzeichnet, dass** das flüssige Lösungsmittel aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt wird.

## Claims

1. A process for the preparation of triethylenediamine (TEDA) by reaction of ethylenediamine (EDA) in the presence of a zeolite catalyst, wherein
the zeolite catalyst comprises one or more metals M in oxidation state IV as oxides, the metal M in oxidation state IV is selected from the group consisting of Ti, Zr, Ge, Hf and Sn, and mixtures thereof,
for M = metal in oxidation state IV or M = two or more metals in oxidation state IV, has an SiO₂ / MO₂ molar ratio of greater than 10 : 1
and the reaction temperature is from 250 to 500°C.

2. The process according to claim 1, wherein the reaction is carried out continuously.

3. The process according to claim 1 or 2, wherein the reaction is carried out in the gas phase.

4. The process according to one of the preceding claims, wherein the reaction is carried out in the presence of a solvent or diluent.

5. The process according to one of the preceding claims, wherein the reaction is carried out in the presence of water and/or ammonia.

6. The process according to one of the preceding claims, wherein the reaction is carried out in the presence of from 2 to 1200% by weight of water, based on EDA employed.

7. The process according to one of the preceding claims, wherein the reaction is carried out in the presence of from 14 to 300% by weight of water, based on EDA employed.

8. The process according to one of the preceding claims, wherein EDA and one or more of the amine compounds from the group consisting of monoethanolamine, diethanolamine, triethanolamine, piperazine (PIP), diethylenetriamine, triethylenetetramine, tri(2-aminoethyl)amine, N-(2-amino-ethyl)ethanolamine, N-(2-hydroxyethyl)-piperazine and N-(2-aminoethyl)piperazine are reacted.

9. The process according to one of the preceding claims, wherein EDA and from 1 to 1000% by weight of piperazine (PIP), based on EDA, are reacted.

10. The process according to one of claims 1 to 8, wherein EDA and from 7 to 250% by weight of piperazine (PIP), based on EDA, are reacted.

11. The process according to one of claims 1 to 8, wherein EDA, from 8 to 250% by weight of PIP and from 23 to 300% by weight of water, in each case based on EDA, are reacted.

12. The process according to one of the preceding claims, wherein PIP arising after the reaction is separated off and fed back into the reaction with EDA.

13. The process according to one of the preceding claims, wherein the consumption of PIP in the overall balance is from 0 to 30 kg per 100 kg of TEDA.

14. The process according to one of the preceding claims, wherein the reaction temperature is from 310 to 390°C.

15. The process according to one of the preceding claims, wherein the absolute pressure is from 0.1 to 10 bar.

16. The process according to one of the preceding claims, wherein the WHSV (weight hourly space velocity), based on the amines employed in the reaction, is from 0.05 to 6 h⁻¹.

17. The process according to one of the preceding claims, wherein the zeolite catalyst, for M = metal in oxidation state IV or M = two or more metals in oxidation state IV, has an SiO₂ / MO₂ molar ratio of from greater than 10 to 40,000 : 1.

18. The process according to one of the preceding claims, wherein the zeolite catalyst is of the pentasil type.

19. The process according to one of the preceding claims, wherein the zeolite catalyst is of the MFI or MEL structural type or mixed structures thereof (MEL/MFI or MFI/MEL).

20. The process according to one of the preceding claims, wherein the zeolite catalyst is treated with a protic acid before use in the process.

21. The process according to one of the preceding claims, wherein the zeolite catalyst has been doped with one or more transition metals.

22. The process according to one of the preceding claims, wherein the zeolite catalyst comprises silicon dioxide as binder.

23. The process according to one of the preceding claims, wherein a zeolite catalyst which has been treated or regenerated in a gas atmosphere in the presence of oxygen or oxygen-supplying substances at a temperature in the range from 250 to 800°C is employed at least partly.

24. The process according to one of the preceding claims, wherein the process is carried out without interruption in at least two reactors connected in parallel, of which in each case one can be decoupled from the starting-material and product stream for regeneration of the zeolite catalyst.

25. The process for the preparation of a solution of TEDA, which comprises preparing TEDA according to one of the preceding claims, evaporating the prepared TEDA, and passing the vapor-form TEDA into a liquid solvent.

26. The process for the preparation of TEDA, which comprises preparing a solution of pure TEDA according to the preceding claim, and subsequently crystallizing the TEDA out of this solution.

27. The process according to one of the two preceding claims, wherein the liquid solvent is selected from the group consisting of cyclic or acyclic hydrocarbons, chlorinated aliphatic hydrocarbons, aromatic hydrocarbons, alcohols, ketones, aliphatic carboxylic esters, aliphatic nitriles and ethers.

## Revendications

1. Procédé de préparation de triéthylènediamine (TEDA) par réaction d'éthylènediamine (EDA) en présence d'un catalyseur de zéolithe, **caractérisé en ce que** le catalyseur de zéolithe contient un ou plusieurs métaux M dans l'étage d'oxydation IV sous forme d'oxydes, le métal M dans l'étage d'oxydation IV est choisi dans le groupe formé par Ti, Zr, Ge, Hf et Sn ou leurs mélanges,
présente, pour M = métal dans l'étage d'oxydation IV ou M = deux métaux ou plus dans l'étage d'oxydation IV, une proportion molaire SiO₂/MO₂ de plus de 10:1, et que la température de réaction est de 250 à 500°C.

2. Procédé selon la revendication 1, **caractérisé en ce que** l'on entreprend la réaction en continu.

3. Procédé selon la revendication 1 ou 2, **caractérisé en ce que** l'on entreprend la réaction dans la phase gazeuse.

4. Procédé selon l'une des revendications qui précèdent, **caractérisé en ce que** l'on entreprend la réaction en présence d'un solvant ou d'un diluant.

5. Procédé selon l'une des revendications qui précèdent, **caractérisé en ce que** l'on entreprend la réaction en présence d'eau et/ou d'ammoniac.

6. Procédé selon l'une des revendications qui précèdent, **caractérisé en ce que** l'on entreprend la réaction en présence de 2 à 1200% en poids d'eau, par rapport à l'EDA mise en oeuvre.

7. Procédé selon l'une des revendications qui précèdent, **caractérisé en ce que** l'on entreprend la réaction en présence de 14 à 300% en poids d'eau, par rapport à l'EDA mise en oeuvre.

8. Procédé selon l'une des revendications qui précèdent, **caractérisé en ce que** l'on fait réagir de l'EDA et un ou plusieurs des composés amines du groupe formé par la monoéthanolamine, la diéthanolamine, la triéthanolamine, le pipérazine (PIP), la diéthylènetriamine, la triéthylènetétramine, la tri (2-aminoéthyl)amine, la N-(2-aminoéthyl)éthanolamine, la N-(2-hydroxyéthyl)pipérazine et la N-(2-aminoéthyl)pipérazine.

9. Procédé selon l'une des revendications qui précèdent, **caractérisé en ce que** l'on fait réagir de l'EDA et de 1 à 1000% en poids de pipérazine (PIP), par rapport à l'EDA.

10. Procédé selon l'une des revendications 1 à 8, **caractérisé en ce que** l'on fait réagir de l'EDA et de 7 à 250% en poids de pipérazine (PIP), par rapport à l'EDA.

11. Procédé selon l'une des revendications 1 à 8, **caractérisé en ce que** l'on fait réagir de l'EDA, de 8 à 250% en poids de PIP et de 23 à 300% en poids d'eau, à chaque fois par rapport à l'EDA.

12. Procédé selon l'une des revendications qui précèdent, **caractérisé en ce que** l'on sépare la PIP présente après la réaction et qu'on la recycle dans la réaction avec l'EDA.

13. Procédé selon la revendication qui précède, **caractérisé en ce que** la consommation de PIP dans le bilan total est de 0 à 30 kg par 100 kg de TEDA.

14. Procédé selon l'une des revendications qui précèdent, **caractérisé en ce que** la température de réaction est de 310 à 390°C.

15. Procédé selon l'une des revendications qui précèdent, **caractérisé en ce que** la pression absolue est de 0,1 à 10 bar.

16. Procédé selon l'une des revendications qui précèdent, **caractérisé en ce que** la vitesse spatiale pondérale horaire (WHSV, weight hourly space velocity) par rapport aux amines mises en oeuvre dans la réaction est de 0,05 à 6 h⁻¹.

17. Procédé selon l'une des revendications qui précèdent, **caractérisé en ce que** le catalyseur de zéolithe présente, pour M = métal dans l'étage d'oxydation IV ou M = deux métaux ou plus dans l'étage d'oxydation IV, une proportion molaire de SiO₂ / MO₂ de plus de 10 à 40 000 : 1.

18. Procédé selon l'une des revendications qui précèdent, **caractérisé en ce que** le catalyseur de zéolithe est du type pentasil.

19. Procédé selon l'une des revendications qui précèdent, **caractérisé en ce que** le catalyseur de zéolithe est du type de structure MFI, MEL ou de leurs structures mixtes (MEL/MFI, MFI/MEL).

20. Procédé selon l'une des revendications qui précèdent, **caractérisé en ce que** le catalyseur de zéolithe est traité par un acide protonique avant la mise en oeuvre dans le procédé.

21. Procédé selon l'une des revendications qui précèdent, **caractérisé en ce que** le catalyseur de zéolithe est dopé par un ou plusieurs métaux de transition.

22. Procédé selon l'une des revendications qui précèdent, **caractérisé en ce que** le catalyseur de zéolithe contient du dioxyde de silicium en tant que liant.

23. Procédé selon l'une des revendications qui précèdent, **caractérisé en ce que** l'on met en oeuvre au moins en partie un catalyseur de zéolithe qui a été traité ou régénéré dans une atmosphère gazeuse en présence d'oxygène ou de substances libérant de l'oxygène, à une température de l'ordre de 250 à 800°C.

24. Procédé selon l'une des revendications qui précèdent, **caractérisé en ce que** le procédé est entrepris sans interruption dans au moins deux réacteurs installés en parallèle, dont l'un peut à chaque fois être désaccouplé du courant d'éduit et de produit aux fins de régénération du catalyseur de zéolithe.

25. Procédé de préparation d'une solution de TEDA, **caractérisé en ce que** l'on prépare de la TEDA selon l'une des revendications qui précèdent, que l'on évapore la TEDA et que l'on envoie la TEDA sous forme de vapeur dans un solvant liquide.

26. Procédé de préparation de TEDA, **caractérisé en ce que** l'on prépare une solution de TEDA selon la revendication qui précède, et que l'on cristallise ensuite la TEDA préparée à partir de cette solution.

27. Procédé selon l'une des deux revendications qui précèdent, **caractérisé en ce que** le solvant liquide est choisi dans le groupe formé par des hydrocarbures cycliques ou acycliques, des hydrocarbures aliphatiques chlorés, des hydrocarbures aromatiques, des alcools, des cétones, des esters d'acides carboxyliques aliphatiques, des nitriles et des éthers aliphatiques.
